# EUROPEAN PATENT APPLICATION

(11) **EP 4 074 320 A1**
(43) Date of publication of application: **19.10.2022**
(21) Application number: 21168183.8
(22) Date of filing: 13.04.2021
(51) Int. Cl.: A61K 35/17, A61P 17/02, A61K 8/98, A61K 8/99, A61Q 19/00

(54) **TREATMENT OF SKIN SCARS**

(71) Applicant: Aposcience AG, 1200 Wien (AT)
(72) Inventor: Mildner, Michael, 3040 Neulengbach (AT); Ankersmit, Hendrik Jan, 1060 Wien (AT)
(74) Representative: Schwarz & Partner Patentanwälte GmbH

(57) **Abstract**

The present invention relates to a composition comprising a supernatant of a peripheral blood mononuclear cell (PBMC) cell culture for use in the treatment of skin scars.

## Description

### TECHNICAL FIELD

The present invention relates to the field of skin scar treatment.

### BACKGROUND ART

Administering conditioned medium from mesenchymal stem cells (SCs) revealed that secreted factors, rather than SCs themselves, exert beneficial paracrine effects and account for most of the initial findings. It has been previously shown that stressed peripheral blood mononucleated cells (PBMCs) represent an easily accessible and rich source for cellular secretomes with strong regenenerative activity (APOSEC). Several modes of action have been recently elucidated and various clinical indications have already been described for APOSEC, including wound healing, acute myocardial infarction, autoimmune myocarditis, cerebral ischemia, and spinal cord injury.

Formation of pathologic scars is a complex medical problem and represents a relevant global disease burden. In the western world, more than 100 million people acquire scars every year, and an estimated number of 11 million people develop keloids. The risk to develop hypertrophic scars following surgery or burn injury is above 30%. In contrast to regular scars, hypertrophic scars are characterised by excessive fibrotic processes within the injured area. By contrast, keloids are progressive fibro-proliferative malformations that form within scar tissue and over-grow the borders of the original injury. The molecular mechanisms leading to formation of hypertrophic scars or keloids are not well understood. Beside posing significant psychological problems, hypertrophic scars and keloids are painful, pruritic and cause movement restrictions. Therapeutic options are still limited and no gold standard for the treatment of pathologic scars exists. Possible options are 1) administration of steroids inhibiting fibroblast proliferation, 2) radiation-induced cell cycle arrest and cell death of fibroblasts, 3) pressure therapy with effects on wound tension and 4) surgical removal of the scarred tissues, which, however, shows extremely high recurrence rates. In addition, chemo-therapeutics and other substances have been used, however, without eminent efficacy. The most promising treatment option so far is a combination of surgical removal and subsequent treatment with steroids.

Thus, it is an object of the present invention to provide methods to remove/treat skin scars which do not require surgery and/or medication having unwanted side effects.

### SUMMARY OF THE INVENTION

The present invention relates to a composition comprising a supernatant of a peripheral blood mononuclear cell (PBMC) cell culture for use in the treatment of skin scars.

It turned surprisingly out, that supernatants of PBMC cell cultures can be used in the treatment of skin scars. Supernatants of PBMC cell cultures are already known to be used in the treatment of damaged tissues like skin wounds (see e.g. EP 2 379 085). However, completely different biological processes and cell types are involved in wound healing as compared to mature skin scars.

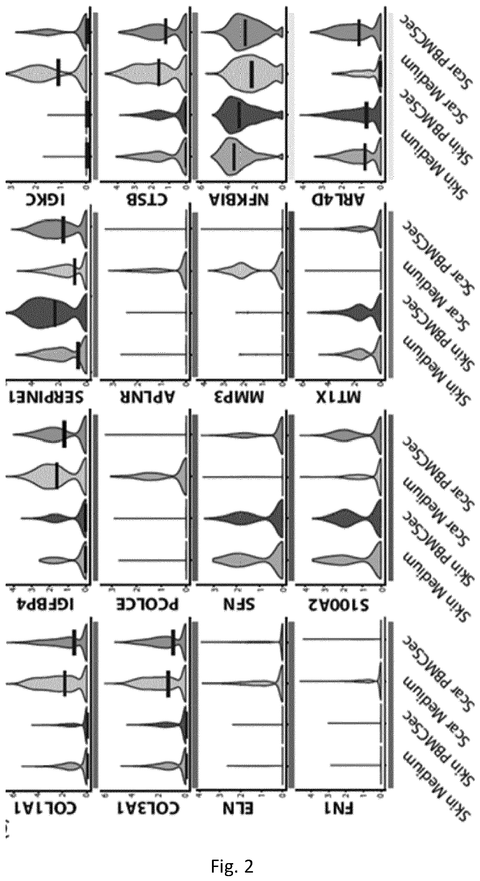

Wound healing is a complex process and consists of 4 phases: the hemostasis phase, the inflammatory phase, the proliferative phase and the maturation phase. Whereas the main function of wound healing is a rapid closure of the damaged area and establishment of a new protective barrier against the environment, a scar is the fibrotic tissue that remains after wound healing, which replaces normal skin. In contrast to normal skin, scar tissue is mainly characterized by an overproduction of extracellular matrix, such as the collagens COL1A1 and COL3A1, elastin (ELN), fibronectin 1 (FN1), and the procollagen (PCOLCE) (Fig 2). Furthermore, several factors modifying the extracellular matrix, including cytokine (e.g. insulin-like growth factor binding protein 4(IGFBP4)), proteases (e.g. matrix metalloproteinase-3 (MMP3)) and protease inhibitors (e.g. Serpin Family E Member 1 (SERPINE1) are significantly deregulated in scar tissue (Fig 2). In addition, myofibroblasts are highly active leading to skin contraction. The main goal of scar treatment is therefore the reduction of myofibroblasts and the overshooting production of components of the extracellular matrix as well as the normalisation of the deregulated production and matrix-modifying factors.

Another aspect of the present invention relates to a method of cosmetic treatment of skin scars comprising the step of administering an effective amount of a composition comprising a supernatant of a peripheral blood mononuclear cell (PBMC) cell culture to skin scars.

### BRIEF DESCRIPTION OF THE FIGURES

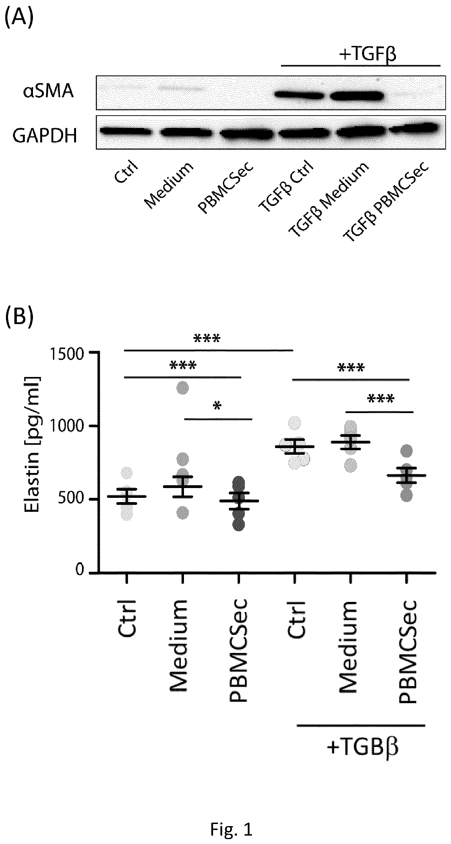

Fig. 1 shows that APOSEC tackles TGF-mediated myofibroblast-differentiation. A) Western blot of primary human skin fibroblast stimulated with Medium or APOSEC in absence or presence of TGFbeta. APOSEC prevented TGFbeta-induced differentiation of fibroblasts into myofibroblasts as shown by the expression of alphaSMA. ELISA of supernatants of TGFbeta stimulated fibroblasts showed enhanced production of elastin. Addition of APOSEC reduced the enhanced secretion of elastin.
Fig. 2 shows scRNAseq of biopsies of human hypertrophic scars and normal skin treated ex vivo with medium or APOSEC. Genes associated with skin fibrosis are shown. Treatment with APOSEC normalized the expression of these genes to values observed in normal skin.

### DESCRIPTION OF EMBODIMENTS

The present invention relates to a composition comprising a supernatant of a PBMC cell culture for use in the treatment of skin scars.

Cells, in particular mammalian cells, are known to secrete numerous substances during cultivation into a cell culture medium. The so obtained conditioned culture media can be used in the treatment and/or prevention of various diseases and disorders. For instance, WO 2010/070105 and WO 2010/079086 disclose conditioned culture media ("supernatants") which are obtained by cultivation of PBMCs and which can be used in the treatment of various inflammatory conditions. Hence, "a supernatant of a peripheral blood mononuclear cell (PBMC) cell culture", as used herein, refers to any supernatant obtainable by cultivating PBMCs *in vitro* in a culture medium. After the cultivation step the cultivated PBMCs are removed from the culture medium in order to obtain the substantially cell-free, preferably completely cell-free, supernatant. The supernatant of the PBMC culture comprises next to components of the cultivation medium substances produced and secreted by the PBMCs and/or even lysed PBMCs. "Supernatant" can be used interchangeable with conditioned culture medium obtainable by cultivating PBMCs.

The supernatant of the present invention is obtainable by cultivating PBMCs, which are subjected to ionizing radiation before or during cultivation. The ionizing radiation is preferably gamma radiation.

A "skin scar", as used herein, refers to a fibrous tissue that replaces normal skin tissue after a skin injury. Skin scars are a result from the biological process of wound repair in the skin. Scar tissue is typically composed of the same protein (i.e. collagen) as the tissue that it replaces. However, the fiber composition in scar tissue differs significantly from that of scar free tissue. In normal tissue collagen forms a random basket weave whereas in scar tissue the collagen is cross-linked and forms an alignment in a single direction. Due to this collagen scar tissue alignment the scar skin is usually of inferior functional quality compared to the normal collagen randomised alignment so that the skin will be, for instance, less resistant to ultraviolet radiation, and sweat glands and hair follicles do not grow back within scar tissues. According to the present invention "skin scar" refers to any type of skin scars and in particular to hypertrophic scars, keloid scars and stretch marks.

A hypertrophic scar is the result of an overproduction of collagen, which causes a scar to be raised, however, within the boundaries of the original wounds. They usually occur within 4 to 8 weeks following wound infection or wound closure.

Keloid scars are a more serious form of excessive scarring, because they can grow indefinitely into large, tumorous (although benign) neoplasms. Keliod scars extend usually outside the original wound area. They can be itchy or painful and their surgical removal may exacerbate the condition and worsening of the keloid.

Stretch marks, also known as striae are also a form of scarring. These are caused when the skin is stretched rapidly (for instance during pregnancy, significant weight gain, or adolescent growth spurts), or when skin is put under tension during the healing process.

The terms "treatment" and "treating" of skin scars, as used herein, refer to the reduction of the scar volume and/or scar size by at least 20%, preferably at least 40%, more preferably at least 50%, more preferably at least 60%, more preferably at least 70%, more preferably at least 80%, more preferably at least 90%, more preferably at least 95%, and/or ideally to the re-establishment of the original tissue. The administration of the composition of the present invention should result in a partial or even complete transformation of scar tissue into normal tissue.

A further aspect of the present invention relates to a method of cosmetic treatment of skin scars comprising the step of administering an effective amount of a composition comprising a supernatant of a peripheral blood mononuclear cell (PBMC) cell culture to skin scars.

According to a preferred embodiment of the present invention the composition of the present invention is applied on a skin scar or injected into a skin scar.

The composition of the present invention can be applied topically on or injected into a skin scar. It turned out that for smaller skin scars a topical administration of the composition of the present invention on the scar is sufficient. In particular stretch marks can be treated topically. If a scar comprises an excessive accumulation of collagen it is advantageous to inject the composition of the present invention into the scar and/or the tissue surrounding the scar.

If the composition of the present invention is topically administered, said composition may be provided as a gel, preferably hydrogel, as an ointment, as a paste, as a cream, as a powder, as a liniment or as a lotion. Oleaginous bases, absorption bases, water in oil emulsion bases, oil in water emulsion bases, water soluble or water miscible bases can be used for the formulation of these topical dosage forms and may also contain anti-oxidants, such as vitamin C and others.

According to another preferred embodiment of the present invention the composition is injected into a skin scar by a needle or a microneedle assembly comprising a plurality of microneedles.

The composition of the present invention can be injected into a skin scar by using needles. Depending on the size of the scar, the composition is injected at one or more injection sites into the scar. Particularly advantageous is the use of microneedle assemblies, comprising a plurality of microneedles. The use of such microneedle assemblies allows to inject the composition of the present invention simultaneously at more than one injections sites. Furthermore, the microneedle assembly is particularly suited to introduce the composition of the present invention into to the skin scar by penetrating the scar which has been previously covered with said composition. In such a case the microneedle assembly is moved into and out of the skin for several times.

The microneedle assembly can also be part of a transdermal patch comprising the composition of the present invention which is applied on a skin scar. Such a transdermal patch is particularly suited because it allows to administer the composition of the present invention continuously over an extended period of time.

According to another preferred embodiment of the present invention wherein the composition is injected into the skin scar at least in part during the removal of the needle or the microneedles.

In order to spread the composition of the present invention more homogenously within the skin scar it is preferred to inject the composition during the removal of the needle or needles from the skin scar.

The skin scar is preferably a hypertrophic scar, a keloid scar or a stretch mark.

According to another preferred embodiment of the present invention the PBMC cell culture comprises monocytes, T cells, B cells and/or NK cells.

According to a further preferred embodiment of the present invention the PBMCs cells are cultivated in a cell culture medium selected from the group consisting of a cell growth medium, preferably CellGro medium, more preferably Cellgro GMP DC medium, RPMI, DMEM, X-vivo and Ultraculture.

The PBMCs of the PBMC cell culture are subjected to ionizing radiation before or during cultivation. In addition to these stress inducing conditions the PBMCs can be subjected to further stress. Hence, According to a preferred embodiment of the present invention the PBMCs are subjected to one or more further stress inducing conditions before or during cultivation.

The term "under stress inducing conditions", as used herein, refers to cultivation conditions leading to stressed cells. Conditions causing stress to cells include among others heat, chemicals, radiation, hypoxia, osmotic pressure etc.

Additional stress to the cells of the present invention leads to a further increase of the expression and secretion of substances beneficial for treating inflammatory skin conditions, in particular skin conditions associated with ischemia.

According to a preferred embodiment of the present invention the stress inducing conditions include hypoxia, ozone, heat (e.g. more than 2°C, preferably more than 5°C, more preferably more than 10°C, higher than the optimal cultivation temperature of PBMCs, i.e. 37°C), radiation (e.g. UV radiation, gamma radiation), chemicals, osmotic pressure (i.e. osmotic conditions which are elevated at least 10% in comparison to osmotic conditions regularly occurring in a body fluid, in particular in blood) or combinations thereof.

Hence, according to a further preferred embodiment of the present invention the stress inducing condition is selected from the group consisting of radiation, in particular ionizing radiation or UV radiation, hypoxia, ozone, heat, osmotic pressure and pH shift.

According to another preferred embodiment of the present invention the PMCs are subjected to an ionizing radiation, preferably gamma radiation, at a dose of at least 10 Gy, preferably at least 20 Gy, more preferably at least 40 Gy, more preferably at least 50 Gy.

According to a preferred embodiment of the present invention the PBMCs are cultivated for at least 4 h, preferably for at least 6 h, more preferably for at least 12 h, before isolating its supernatant.

According to a preferred embodiment of the present invention the PCBMC cell culture comprises 1x10⁵ to 1x10⁸ PBMCs/ml, preferably 1x10⁶ to 1x10⁷ PBMCs/ml, more preferably 2x10⁶ to 5x10⁶ PBMCs/ml.

The composition of the present invention may comprise pharmaceutically acceptable excipients such as diluents (for example buffers like phosphate-buffered saline (PBS), NaCl solutions or Hank's balanced salt solution (HBSS)), stabilizers (for example butylated hydroxytoluene (BHT), butylated hydroxyanisole (BHA), vitamin C, vitamin E), carriers etc. Methods for preparing the same are well known to the skilled artisan.

In order to increase the shelf-life of the composition according to the present invention the supernatant may be lyophilised. Methods for lyophilising such preparations are well known to the person skilled in the art.

Prior its use the lyophilised preparation can be contacted with water or an aqueous solution comprising buffers, stabilizers, salts etc.

According to another preferred embodiment of the present invention the composition of the present invention can be used to treat skin scars of humans and all kind of mammals such as horses, dogs, cats, and camels.

The present invention is further illustrated by the following example without being restricted thereto.

### EXAMPLE

### Material & Methods

### Patient material

Resected scar tissue was gained from three patients who underwent elective scar resection surgery. Scars were previously classified as hypertrophic, pathological scars according to POSAS (Fearmonti RM et al. Plastic and reconstructive surgery 127, 242 (2011)) by a plastic surgeon. All scars were mature scars, i.e. at least two years old, were not operated on and were previously not treated with corticosteroids, 5-FU, irradiation or similar. All scar samples were obtained from male and female patients younger than 45 years, with no chronic diseases or chronic medication. Healthy skin was obtained from three healthy female donors between 25-45 years from surplus abdominal skin removed during elective abdominoplasty.

### Animals

In all animal experiments, 8-12 weeks old female Balb/c mice (Medical University of Vienna Animal Breeding Facility, Austria) were used. Mice were housed according to enhanced standard husbandry at a 12/12h light/dark cycle in a selected pathogen-free environment, with food and water ad libidum.

### Full thickness wound and scarring model in mice

For a full thickness skin wound and scarring model, mice were deeply anesthetized with ketamine 80-100 mg/kg xylazine 10-12.5 mg/kg i.p., and postoperative analgesia by s.c. injection of 0,1ml/10mg Buprenophin and Piritramid 7,5 mg/ml in drinking water. A 9x9mm square area was marked on the back and excised with sharp scissors. The wounds were left to heal uncovered without any further intervention for 4 weeks, and the resulting scar tissue was observed and documented.

### Production of APOSEC

Secretomes of PBMCs were produced in compliance with good manufacturing practice (GMP) by the Austrian Red Cross, Blood Transfusion Service for Upper Austria (Austria) as described (Wagner T et al. Sci Rep 8, 18016 (2018); Laggner M et al. Stem cell research & therapy 11, 9 (2020)). PBMCs were obtained by Ficoll-Paque PLUS (GE Healthcare, USA)-assisted density gradient centrifugation, adjusted to a concentration of 25×10⁶ cells/mL (25U/ml, 1 Unit= secretome of 1 Million cells) and exposed to 60 Gy Caesium 137 gamma-irradiation (IBL 437C, Isotopen Diagnostik CIS GmbH, Germany). Cells were cultured in phenol red-free CellGenix GMP DC medium (CellGenix GmbH, Germany) for 24 ± 2 hours. Cells and cellular debris were removed by centrifugation and supernatants were passed through a 0.2 µm filter. For viral clearance, methylene blue treatment was performed as described (Gugurell A et al, Blood Transfus 18(2020):30-39). Secretome was lyophilized, terminally sterilized by high-dose gamma-irradiation, and stored at -80°C. All experiments were performed using secretomes produced under GMP of the following batches: A000918399086, A000918399095, and A000918399098, A000918399101, A000918399102, and A000918399105. Immediately before performing experiments, lyophilizate was resuspended in 0,9% NaCl to the original concentration of 25U/ml.

### APOSEC stimulation of mouse scars

Starting on day 29 after skin wounding, mice were injected with 100yl 0,9% NaCl, medium (CellGenix GMP DC Medium phenol red-free), or APSEC, prepared as described above, every second day for two weeks. Subsequently, half of the mice from each group were sacrificed and brought to analysis, the other half were left for another two weeks without further intervention, and then sacrificed.

### Ex vivo skin and scar stimulation

From human skin and scar tissue, 6mm punch biopsies were taken, subcutaneous adipose tissue was removed and biopsies placed in 12-well plates supplemented with 400yl DMEM (Gibco, Thermo Fisher, USA, with 10% fetal bovine serum and 1% penicillin/streptomycin), and 100µl CellGenix medium or 100yl APOSEC. In addition, 100µl medium or APOSEC were injected into upper dermis in the middle of the biopsy. Biopsies were incubated for 24h and then harvested for scRNAseq analysis.

### Skin and scar APOSEC stimulation, cell isolation and droplet-based scRNAseq

Mouse scars and human stimulated skin and scar samples were digested in Miltenyi Whole Skin dissociation Kit (Miltenyi Biotec, Germany) for 2.5h according to the manufacturer's protocol and processed on a GentleMACS OctoDissociator (Miltenyi). The cell suspension was processed through a 100µm and a 40µm filter, centrifuged for 10min at 1500rpm, washed twice and resuspended in 0.04% FBS in phosphate buffered saline (PBS). DAPI 1µl/1 Million cells was added for 30sec, cells were again washed twice and were sorted for viability on a MoFlo Astrios high speed cell sorting device (Beckman-Coulter, USA), and only distinctly DAPI-negative cells were used for further processing. Immediately after sorting, viable cells were loaded on a 10X-chromium instrument (Single cell gene expression 3'v 2/3, 10X Genomics, USA) to generate a Gel-bead in emulsion (GEM). GEM-generation, library preparation, RNA-sequencing, demultiplexing and counting were done by the Biomedical Sequencing Core Facility of the Center for Molecular Medicine (CeMM, Austria). Sequencing was performed 2x75 bp, paired end on an Illumina HiSeq 3000/4000 (Illumina, USA).

### Results

### APOSEC prevents TGFbeta-mediated breakdown of dermal elastic fibers

Ex vivo biopsies were injected with TGFbeta1, TGFbeta1 and medium and TGFbeta1 and APOSEC. Biopsies of normal human skin were injected with TGFbeta1 to induce scar like tissue remodeling. Two days after injection of TGFbeta1 a compact extracellular matrix (ECM) and a breakdown of elastic fibers was observed. Similar morphological alterations were observed when TGFbeta1 and medium were injected into the skin. A combination of TGFbeta1 with APOSEC inhibited ECM deposition and degradation of elastic fibers.

### APOSEC prevents TGFbeta-induced ECM-accumulation in mouse skin

Next, it was tested whether similar effects were observed when APOSEC was injected into mouse skin *in vivo.* The analyses showed that APOSEC significantly reduced collagen production and deposition after injection of TGFbeta1. In addition a reduced expression of alpha-smooth muscle actin was observed, representing myofibroblasts.

### APOSEC prevents TGFbeta-induced differentiation of fibroblasts to myofibroblasts.

To further investigate the effect on differentiation of fibroblasts to contractile myofibroblasts human primary fibroblasts were stimulated *in vitro* with TGFbeta. Stimulation with TGFbeta strongly induced expression of alpha-smooth muscle actin (alphaSMA), a protein marker for myofibroblasts. While addition of medium alone had no effect on alphaSMA expression, addition APOSEC completely abolished TGFbeta induced differentiation of fibroblasts to myofibroblasts (Fig. 1A). In addition, protein expression of elastin was significantly induced by TGFbeta and inhibited by APOSEC (Fig. 1B)

### Intradermal injection of APOSEC in human scars ex vivo regulate RNA-expression of scar associated genes

Punch biopsies of human skin and scar tissue were intradermally injected with APOSEC or control medium. 24 hours later biopsies were enzymatically dissociated and single cells were analyzed by single cell RNA sequencing (Fig. 2). Several mRNAs, associated with scar formation, showed significant regulation after injection of APOSEC. The administration of APOSEC into scar tissue clearly changed the mRNA profile to those levels which occur in healthy skin (i.e. non-scar skin).

### Conclusion

These analyses show that the administration, in particular the intradermal injection, of APOSEC is able to improve scar quality by inhibiting myofibroblast differentiation and production and deposition of extracellular matrix components. APOSEC clearly improves already existing mature scars (in contrast to the new development of scars).

## Claims

1. Composition comprising a supernatant of a peripheral blood mononuclear cell (PBMC) cell culture for use in the treatment of skin scars.

2. A method of cosmetic treatment of skin scars comprising the step of administering an effective amount of a composition comprising a supernatant of a peripheral blood mononuclear cell (PBMC) cell culture to skin scars.

3. Composition for the use according to claim 1 or method according to claim 2, wherein the composition is applied on a skin scar or injected into a skin scar.

4. Composition for the use according to claim 3 or method according to claim 3, wherein the composition is injected into a skin scar by a needle or a microneedle assembly comprising a plurality of microneedles.

5. Composition for the use according to claim 4 or method according to claim 4, wherein the composition is injected into the skin scar at least in part during the removal of the needle or the microneedles.

6. Composition for the use according to any one of claims 1 or 3 to 5 or method according to any one of claims 2 to 5, wherein the skin scar is a hypertrophic scar, a keloid scar or a stretch mark.

7. Composition for the use according to any one of claims 1 or 3 to 6 or method according to any one of claims 2 to 6, wherein the PBMC cell culture comprises monocytes, T cells, B cells and/or NK cells.

8. Composition for the use according to any one of claims 1 or 3 to 7 or method according to any one of claims 2 to 7, wherein the PBMCs are cultivated in a cell culture medium selected from the group consisting of a cell growth medium, preferably CellGro medium, more preferably Cellgro GMP DC medium, RPMI, DMEM, X-vivo and Ultraculture.

9. Composition for the use according to any one of claims 1 or 3 to 8 or method according to any one of claims 2 to 8, wherein the PBMCs are subjected to one or more stress inducing conditions before or during cultivation.

10. Composition for the use according claim 9 or method according to claim 9, wherein the stress inducing condition is selected from the group consisting of radiation, in particular ionizing radiation or UV radiation, hypoxia, ozone, heat, osmotic pressure and pH shift.

11. Composition for the use according claim 10 or method according to claim 10, wherein the PMCs are subjected to an ionizing radiation at a dose of at least 10 Gy, preferably at least 20 Gy, more preferably at least 40 Gy, more preferably at least 50 Gy.

12. Composition for the use according to any one of claims 1 or 3 to 11 or method according to any one of claims 2 to 11, wherein the PBMCs are cultivated for at least 4 h, preferably for at least 6 h, more preferably for at least 12 h, before isolating its supernatant.

13. Composition for the use according to any one of claims 1 or 3 to 12 or method according to any one of claims 2 to 12, wherein the PCBMC cell culture comprises 1x10⁵ to 1x10⁸ PBMCs/ml, preferably 1x10⁶ to 1x10⁷ PBMCs/ml, more preferably 2x10⁶ to 5x10⁶ PBMCs/ml.
